# EUROPEAN PATENT SPECIFICATION

(11) **EP 1 922 988 B1**
(45) Date of publication and mention of the grant of the patent: **08.04.2015**
(21) Application number: 06124382.0
(22) Date of filing: 20.11.2006
(51) Int. Cl.: A61B 5/00, A61B 5/0215

(54) **Transceiver unit in a pressure measurement system**
Sendeempfängereinheit in Druckmesssystem
Unité émetteur-récepteur dans un système de mesure de la pression sanguine

(43) Date of publication of application: 21.05.2008
(73) Proprietor: St. Jude Medical Systems AB, 751 35 Uppsala (SE)
(72) Inventor: Samuelsson, Magnus, 18230 Danderyd (SE); Tulkki, Sauli, 83110 Phuket (TH)
(74) Representative: Brann AB

(56) References cited:
- WO-A-93/09837
- WO-A-03/075744
- US-A1- 2003 141 916
- US-A1- 2006 009 817
- US-B1- 6 248 080
- US-B1- 6 428 336

## Description

### Field of the invention

The present invention relates to a transceiver unit and a pressure measurement system for measuring a physiological variable in a body.

### Background of the invention

In many medical procedures, medical personnel need to monitor various physiological conditions that are present within a body cavity of a patient. These physiological conditions are typically physical in nature - such as pressure, temperature, rate-of-fluid flow - and provide the physician or medical technician with critical information as to the status of a patient's condition. Obviously, the manner by which these types of parameters are measured and monitored must be safe, accurate and reliable.

One device that is widely used to monitor such conditions is the blood pressure transducer. A blood pressure transducer senses the magnitude of a patient's blood pressure, and converts it into a representative electrical signal. This electrical signal is then supplied to a vital signs monitor that displays, records or otherwise monitors the magnitude of the patient's blood pressure.

Traditionally, a blood pressure transducer has consisted of a pressure responsive diaphragm that is mechanically coupled to piezoresistive elements connected in a Wheatstone Bridge-type circuit arrangement. When the diaphragm is placed in fluid communication with a body cavity (such as within the arterial or venous system), pressure induced deflections of the diaphragm cause the resistive elements to be stretched (or compressed, depending on their orientation). According to well-known principles, this alters the resistance of the elements in a manner that is proportional to the applied pressure. The magnitude of the applied pressure can thus be detected by applying an excitation power signal (usually in the form of a voltage) to the inputs of the Wheatstone bridge circuit, and by simultaneously monitoring the bridge output signal. The magnitude of that signal reflects the amount by which the bridge resistance has changed, according to Ohm's law.

Typically, an electrical cable connects the Wheatstone bridge portion of the transducer sensor to a transducer amplifier circuit contained within the vital signs monitor. This amplifier circuit supplies the excitation power signal to the Wheatstone bridge, and simultaneously monitors the bridge output signal. The excitation power signal is typically in the form of a voltage and, depending on the monitor type and manufacturer, can have varying magnitudes and formats, both time-varying (sinusoidal, square-waved and pulsed) and time independent (DC).

According to the principles under which conventional Wheatstone bridge transducers operate, transducer amplifier circuits in most patient monitors have been designed to expect a sensor output signal having a magnitude that is proportional to the magnitude of the excitation power signal and also proportional to the magnitude of the sensed pressure. Because different monitors supply excitation power signals having different magnitudes and/or frequencies, standard proportionality constants have been developed. These proportionality standards allow any sensor to be readily adapted for use with any patient monitor also calibrated to adhere to the proportionality standard.
Several benefits are provided by this compatibility. Blood pressure transducers could be used interchangeably with patient monitors from different manufacturers. As such, medical personnel were not required to select a specific transducer for use with a specific monitor. Further, hospital investments in pre-existing patient monitors were preserved, thereby reducing costs. As a consequence, vital signs monitors adhering to these proportionality standards have achieved almost universal acceptance in medical environments.

However, the blood pressure transducers and monitors that have been previously used, and the resulting standards that have evolved, are not without drawbacks. For instance, the sensors used in these systems were typically positioned external to the patient's body and placed in fluid communication with the body cavity via a fluid-filled catheter line. Pressure variations within the body cavity are then indirectly communicated to the diaphragm by way of fluid contained with the catheter line. As such, the accuracy of such systems has suffered due to variations in hydrostatic pressure and other inconsistencies associated with the fluid column.

In response to this problem, miniaturized sensors using advanced semiconductor technologies have been developed. These types of transducer sensors are extremely accurate, inexpensive and still utilize the well known Wheatstone bridge-type of circuit arrangement, which typically, at least partly, is fabricated directly on a silicone diaphragm. Further, the sensors are sufficiently small such that they can actually be placed on the tip of an insertable guide wire and reside directly within the arteries, tissues or organs of the patient. This eliminates the need for a fluid line because the fluid pressure is communicated directly to the transducer diaphragm. As a result, these sensors - often referred to as guide wire-tipped transducers - provide a much more accurate measurement of the patient's blood pressure.

Unfortunately, the electrical configurations of these miniaturized semiconductor sensors are not always compatible with the transducer amplifiers in existing patient monitors. For instance, the miniaturized sensors often cannot operate over the entire range of excitation signal magnitudes and frequencies found among the various types of patient monitors. Thus, they cannot be connected directly to many of the patient monitors already in use. To be used with such existing monitors, a specialized interface must be placed between the sensor and the monitor. Such an arrangement necessitates additional circuitry on the interface and, because existing monitors have been designed to provide only limited amounts of power, the additional circuitry may require an independent source of electrical power. As a consequence, use of the newer miniaturized sensors often adds cost and complexity to the overall system.

In addition, because of the above limitations, these sensors must often be configured to generate an output signal which is proportional to the pressure sensed, but that is not related to the excitation signal, supplied to the sensor by the monitor, in a way that is directly usable by the physiology monitor, e.g. the sensitivity may be different. As discussed, this does not conform with the electrical format required by the many monitors that are commercially available and already in widespread use. As such, the newer sensors can only be used with specific monitor types, thereby requiring additional, and often redundant, equipment to be purchased. This is especially undesirable given the cost sensitivities so prevalent in today's health care environment.

The Association for the Advancement of Medical Instrumentation ("AAMI") has defined power requirements for physiology monitors and in particular the input/output connector to a sensor wire assembly must comply with the standard set by American National Standards Institute ("ANSI")/AAMI BP22-1994 (referred to as "BP22" in the following).

According to the BP22-standard an input/output connector arranged at the proximal end of a five line connector cable includes a pair of differential output signal lines. The output signal lines are driven by a sensor adapting circuitry's output digital to analog converters (discussed further herein below). The differential output signal, by way of example, operates at 5 µV /mmHg / V_{EXC}. An operation range of -150 µV/V to 1650 µV/V therefore represents a sensed pressure range of -30 to 330 mmHg. An exemplary resolution (minimum step) for the differential output signal is 0,2 mmHg.

US-5,568,815 discloses an interface circuit for interfacing a sensor to a patient monitor. The interface circuit includes a power supply circuit that receives an excitation power signal generated by the patient monitor, and derives therefrom unregulated and regulated supply voltages for use by the electrical components on the interface circuit. Further, the power supply circuit generates an appropriate sensor excitation signal. The interface circuit further includes receiving circuitry for receiving a sensor output signal generated by the sensor. A scaling circuit then scales that signal into a parameter signal that is proportional to the physiological condition detected by the sensor, and that is also proportional to the excitation power signal generated by the patient monitor.
An obvious drawback of the device of US-5,568,815 is that, in order to connect the sensor to the monitor, a separate additional unit in the form of the interface circuit is required.

Furthermore, in US-5,568,815 is also discussed the issues of having an electrically conducted device such as a pressure transducer connected both to a patient and to an electronic monitoring instrument. Great care must then be taken to insure that electrical currents at standard power line frequencies cannot flow from the patient, through the transducer connection, and to ground. An additional risk occurs in patients which are undergoing defibrillation while having an electrically conductive transducer attached.

Thus, the insulation problem has previously been addressed by using fiber-optics or opto-isolator devices to achieve the connection with the monitor device.

The physical connection between the sensor device and the monitor device must be seen in the total set-up during pressure measurements which also may include other instruments involved having its cables or connections which may result in a complex and non-user-friendly environment for the user. In this connection also the sterilisation issue must be mentioned; in the systems according to the prior art there are physical connections, irrespectively if it is for electrical or optical communication purposes, directly to the monitoring device, which require that the entire system must be sterilized and eventually disposed.

A solution to the insulation problem is to use wireless communication to transmit the measure values from the sensor to the monitoring device.

In US patent application 2006/0009817, a system and a method for obtaining a wireless communication of physiological variables are disclosed. The system comprises a control unit providing a communication interface preferably for radio frequency communication using a carrier signal, which is generated by a monitoring device. The control unit is arranged with a modulator for modulating the carrier signal with a signal representing a measured physiological value received from a sensor disposed in the body. Thus, the function of the control unit is dependant upon the generation of a carrier signal from an external unit in order to be able to transfer the measured variables.

Furthermore, the above-mentioned US patent application only indicates that the control unit may be attached to the core wire of the guide wire via a connection wire using a suitable connector means, such as a crocodile clip-type connector, or if the connection wire is omitted, directly connecting the core wire to the control unit by a suitable connector. The connector is not further discussed in the above application.

Furthermore, WO 93/09837 discloses an apparatus according to the preamble of claim 1.

Thus, in the complex environment of an operating room and taken the different drawbacks of the prior art solutions, the general object of the present invention is to achieve an improved device being more user-friendly and reliable than the presently available systems.

### Summary of the invention

The above-mentioned object is achieved by the present invention as defined by independent claim 1.

Preferred embodiments are set forth in the dependent claims.

In particular the present invention obviates the need of a physical connection between the patient and the monitoring device by arranging a reliable wireless link connection between an easy-to-use transceiver unit and a communication unit, and in particular that the measured pressure data is generated by the transceiver unit and transferred as a data stream. The transceiver unit, when receiving pressure sensor data from the pressure sensor, is adapted to self-contained, directly or at a later time, generate a wireless transmission of pressure data to the communication unit without using any carrier wave from the communication unit or any other unit.

The communication unit is adapted to be connected to an external device by a standard input/output connector in accordance with an established standard or in accordance with relevant parts of an established standard, e.g. BP22 or USB, as briefly discussed in the background section.

### Short description of the appended drawings

Preferred embodiments of the present invention will be described in detail in the following with reference made to accompanying drawings, in which:
Figure 1 shows an exemplifying sensor mounted on a guide wire in accordance with prior art and which is applicable herein.
Figure 2 schematically illustrates a pressure measurement system according to the present invention.
Figure 3 shows a block diagram schematically illustrating a transceiver unit according to a preferred embodiment of the present invention.
Figure 4 shows a block diagram schematically illustrating a transceiver unit including a sensor signal adapting circuitry according a preferred embodiment of the present invention Figure 5 shows a block diagram schematically illustrating a communication unit.

### Detailed description of preferred embodiments of the invention

In the prior art, it is known to mount a sensor on a guide wire and to position the sensor via the guide wire in a blood vessel in a living body to detect a physical parameter, such as pressure or temperature. The sensor includes elements that are directly or indirectly sensitive to the parameter. Numerous patents describing different types of sensors for measuring physiological parameters are owned by the applicant of the present patent application. For example, temperature could be measured by observing the resistance of a conductor having temperature sensitive resistance as described in US-6,615,067. Another exemplifying sensor may be found in US-6,167,763, in which blood flow exerts pressure on the sensor which delivers a signal representative of the exerted pressure.

In order to power the sensor and to communicate signals representing the measured physiological variable to an external physiology monitor, one or more cables or leads for transmitting the signals are connected to the sensor, and are routed along the guide wire to be passed out from the vessel to the external physiology monitor, conventionally via physical cables. In addition, the guide wire is typically provided with a central metal wire (core wire) serving as a support for the sensor and (optionally) also as an electrical connection to the sensor, and a surrounding tubing. Hence, a guide wire typically comprises a core wire, leads and a protective tubing.

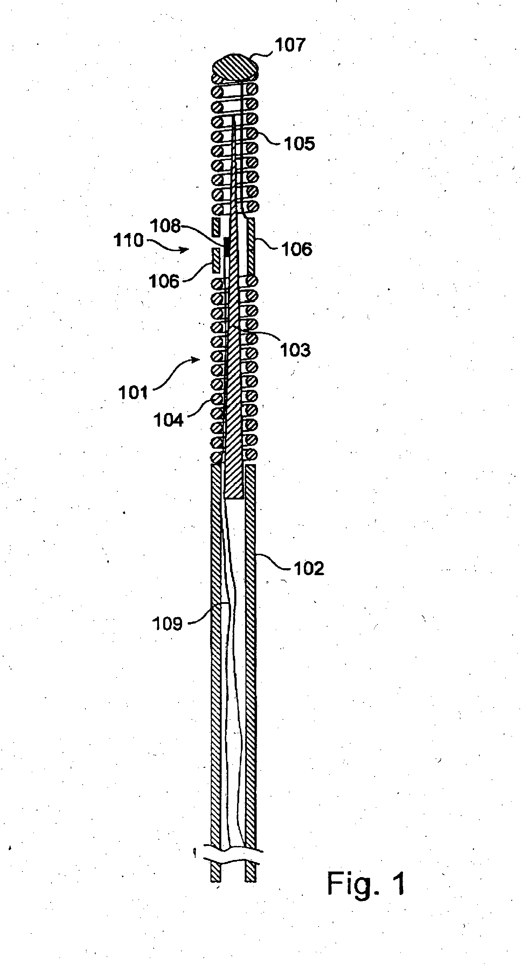

Fig. 1 shows an exemplifying sensor mounted on a guide wire in accordance with conventional design which is applicable for the present invention. The sensor guide wire 101 comprises a hollow tube 102, a core wire 103, a first spiral portion 104, a second spiral portion 105, a jacket or sleeve 106, a dome-shaped tip 107, a sensor element or chip 108, and one or several electrical leads 109. The tube 102 has typically been treated to give the sensor guide construction a smooth outer surface with low friction. The proximal end of the first spiral portion 104 is attached to the distal end of the hollow tube 102, while the distal end of the first spiral portion 104 is attached to the proximal end of the jacket 106. The proximal end of the second spiral portion 105 is connected to the distal end of the jacket 106, and the dome-shaped tip 107 is attached to the distal end of the second spiral portion 105. The core wire 103 is at least partly disposed inside the hollow tube 102 such that the distal portion of the core wire 103 extends out of the hollow tube 102 and into the second spiral portion 105. The sensor element 108 is mounted on the core wire 103 at the position of the jacket 106, and is connected to an external physiology monitor (not shown in the Fig. 1) via the electrical leads 109. The sensor element 108 comprises a pressure sensitive device in the form of a membrane (not shown in the Fig. 1), which through an aperture 110 in the jacket 106 is in contact with a medium, such as blood, surrounding the distal portion of the sensor guide wire 101.

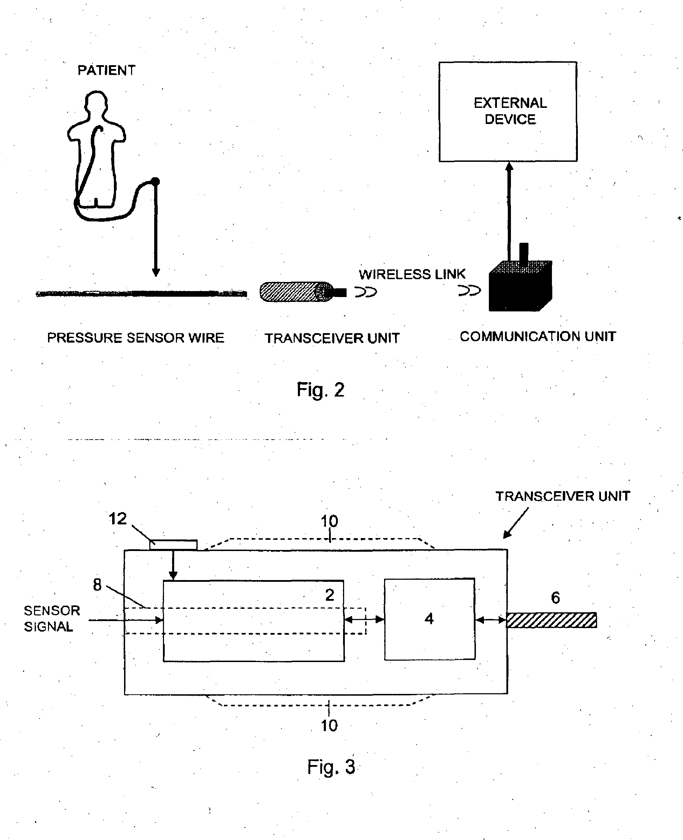

Figure 2 is a schematic overview illustrating the application of the present invention. The pressure measurement system according to the present invention comprises a pressure sensor wire with a pressure sensor to measure pressure inside a patient, and to provide measured pressure data to an external device. The pressure sensor wire is adapted to be connected, at its proximal end, to a transceiver unit adapted to wirelessly communicate via a radio frequency signal with a communication unit arranged in connection with an external device (also referred to as external physiology monitor), in order to transfer measured pressure data to the external device.

The external device may be a dedicated device, i.e. a patient monitoring device, preferably provided with a monitor, or a PC provided with relevant software and external connections to receive and to process the measured data from the pressure measurement system. One example of a dedicated device applicable herein is disclosed in US-6,565,514.

Figure 3 shows a block diagram schematically illustrating the transceiver unit according to the present invention. As shown in figure 1 the transceiver unit is adapted to be connected to the proximal end of a pressure sensor wire provided, at its distal end, with a pressure sensor to measure pressure inside a patient. The transceiver unit comprises a sensor signal adapting circuitry 2, which will be described in greater detail below, a communication module 4, connected to the adapting circuitry 2, that will handled the wireless communication with the communication unit.

In particular during the specific situation where a number of transceiver units are arranged to communicate with one communication unit, also single-directional communication may be used, primarily for the sake of obtaining a reliable communication link.

The measured pressure data is independently generated by the transceiver unit and transferred as a data stream to the communication unit at a prescribed frequency range (in the case where the communication signal is a radio frequency signal), to be further discussed below.

According to a preferred embodiment the communication signal is a radio frequency signal, and that embodiment will be described in detail below.

Furthermore, according to the preferred embodiment the radio frequency signal transmits the data as data packets, i.e. in a digital form. The radio frequency transmission may, as an alternative, be an analogue data transmission.

Generally the communication signal may be an electromagnetic wave signal, e.g. a radio frequency signal, an infrared signal, or a light signal.

According to alternative embodiments the communication signal may be any wirelessly transmitted signal, e.g. an ultrasound signal or a magnetic signal. A person skilled in the art may easily adapt the described system, i.e. the transceiver unit and communication unit, to use any of the mentioned communication signals.

The preferred embodiment where the communication signal is a radio frequency signal will now be described in detail. Although the transceiver unit and the communication unit are described in connection with the preferred embodiment it should be appreciated that relevant features would be equally applicable in case any of the alternative communication signals is used.

With references to figures 2 and 3, the communication module is connected to an antenna 6. In the figures the antenna is illustrated as protruding outside the transceiver unit but may, as in an alternative, be integrated into the housing of the transceiver unit. The pressure sensor wire is adapted to be inserted into an elongated aperture 8 of the transceiver unit. The aperture is at its inner surface provided with a number of electrical connecting surfaces (not shown) to be connected to electrode surfaces at the proximal end of the pressure sensor wire when inserted into the aperture 8. The transceiver unit is further provided with wire fastening means (not shown) to firmly fixate the wire when correctly inserted into the aperture.

According to a preferred embodiment the transceiver unit is adapted to receive the proximal end to the pressure sensor wire having an outer diameter of 0,35 mm, i.e. the inner diameter of the elongated aperture 8 is slightly larger than 0,35 mm.

US-5,938,624 relates to a male connector with a continuous surface for a guide wire which preferably is applied as male connector for the proximal end of the pressure sensor wire to be connected to a transceiver unit according to the present invention. The male connector includes a core wire, and conductive members spaced apart longitudinally along the core wire. A continuous insulating material is disposed between the guide wire and the conductive members and the insulating material having an outer surface coextensive with outer surfaces of the conductive members.

As mentioned above, the transceiver unit according to the present invention is provided with a fastening means to fasten the proximal end of the pressure wire to the transceiver unit. The fastening means may be a female connector of the type disclosed in US-6,428,336 into which a male connector of the kind described above may be inserted and secured to provide electrical contact with contact surfaces of the male connector. The female connector comprises an insulating hollow housing containing three hollow contact members to make contact with the conductive members of the male connector. At the distal end of the female connector the fastening means for securing the male connector in the female connector are provided.

The male connector of the pressure sensor wire used in respect of the present invention is preferably compatible with the female connector disclosed in US-6,428,336.

When the pressure sensor wire is fixated to the transceiver unit the unit may be used as a "handle" when guiding the pressure sensor wire during insertion into a patient. Preferably the transceiver unit is provided with guiding means 10, e.g. in the form of one or many elongated ribs on the outer surface of the transceiver unit, or by providing the transceiver unit with a roughened surface.

The pressure sensor wire is fixated to the transceiver unit such that as the transceiver unit is rotated along its longitudinal axis the sensor wire is also rotated, which often is necessary in order to guide the sensor wire during the insertion procedure.
The transceiver unit is preferably activated and initiated via an activation button 12 arranged at the housing of the unit. The activation button is preferably mechanically activated.

According to an alternative embodiment the transceiver unit is activated and initiated when the proximal end to the sensor wire is correctly inserted into the unit. This may e.g. be achieved by arranging a push button at the bottom of the cavity into which the pressure wire is inserted.

According to another alternative embodiment the transceiver unit is activated and initiated when electrical connections are established between corresponding electrical contact surfaces of the female and male connectors, respectively.

According to still another alternative embodiment the transceiver unit is activated and initiated by a remote signal generated from the communication unit in response of a command from the monitoring device.

The transceiver unit comprises energy means to energize the transceiver unit and the circuitry of the connected pressure sensor wire. The energy means is preferably a battery or a capacitor that e.g. may be included in the sensor signal adapting circuitry.

The pressure sensor wire as well as the transceiver unit are preferably disposable units that must be able to sterilise prior use.

According to an alternative embodiment of the invention, in order to further improve the user-friendliness of the transceiver unit, an attachment means is provided at the housing of the unit. The attachment means may be in the form of a strap, a clip or a hook, i.e. any mechanical attachment means enabling the transceiver unit to be stationary during use.

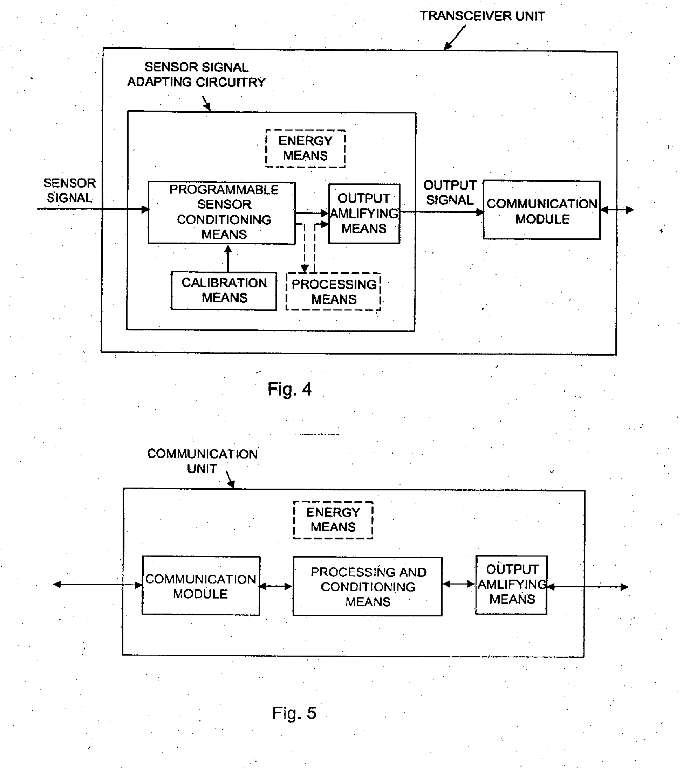

Figure 4 shows a block diagram schematically illustrating a sensor signal adapting circuitry applicable in the present invention and integrated into the transceiver unit.

With references to figures 1 and 2 the pressure sensor wire comprises a sensor element for measuring the physiological variable and to generate a sensor signal in response of said variable, a guide wire having said sensor element at its distal portion, preferably close to its distal end, and adapted to be inserted into the body in order to position the sensor element within the body. The transceiver unit comprises the sensor signal adapting circuitry (fig. 4), wherein the sensor signal is applied to the adapting circuitry that is adapted to automatically generate an output signal, related to the sensor signal, in a format such that the measured physiological variable is retrievable by an external external device. According to a preferred embodiment the sensor signal adapting circuitry comprises a programmable sensor conditioning means, a calibration means, being a storage means into which calibration data may be supplied, stored and altered, e.g. an electrically erasable programmable read-only memory (EEPROM), energy means and an output amplifying means.

The programmable sensor conditioning means is preferably a PGA309 programmable analog sensor conditioner (available from Texas Instruments Inc.) specifically designed for bridge sensors.

According to a preferred embodiment of the present invention the external device supplies the sensor signal adapting circuitry with a reference voltage value wirelessly via the radio link and the corresponding voltage is applied from the energy means in the transceiver unit. By considering the signal standard with which the external device complies, which is indicated to the adapting circuitry by means of the reference voltage, and the actual value of the physical parameter measured by the sensor element, the signal adapting circuitry will process the signal from the sensor element such that an adapted signal in accordance with the standard expected by the monitor may be wirelessly sent back to the external device.

The communication between the transceiver unit and the communication unit is preferably performed in a so-called license-free radio frequency interval. The term "license-free radio" refers to the permission granted by government agencies to allow multiple radios to operate in a specified frequency band at one time. These license-free bands are known as the ISM (Industrial, Scientific and Medical) bands.

The two most commonly used ISM bands are referred to as the 900MHz and the 2.4GHz bands. Both permit the use of license-free Spread Spectrum radios within them. For the most part, the 900MHz band is used in the Americas. The 2.4GHz band is used (with differing power constraints) throughout most of the world. While there are some differences between the characteristics of the two bands, the 900MHz band typically allows for higher power and longer distance transmissions while the 2.4GHz band, with its wider bandwidth, allows for higher data rates. In Europe, the 869 MHz and 433 MHz bands are also classified as ISM bands and China has opened the 220MHz band to license-free radios.

In an embodiment of the present invention a frequency band of 2,4 GHz (2,2 - 2,6 GHz)is used. A typical communication distance would be less than 10 meters.

In order to achieve a secure transmission of the sensor values from the transceiver unit to the communication unit, preferably a frequency hopping technique is used, e.g. by use of Bluetooth. The frequency hopping technique is well-known to persons skilled in the art of radio communication, and will therefore only briefly be described herein.

The transceiver unit comprises a first communication module to handle the radio frequency communication with the communication unit that is provided with a second communication module.

When the pressure sensor wire has been inserted into the transceiver unit and the communication unit is connected to the external device the system is ready for use.
By pressing the activation button on the transceiver unit it is activated and will then try to establish a radio link connection with the communication unit. This is preferably performed by a conventional handshake procedure in order to identify the transceiver unit. The system is now ready to receive measured sensor data.

Pressure sensor values measured at specific times, exemplary designated 1, 2, 3, 4, 5, etc., are respectively designated P1, P2, P3, P4, P5, etc, are applied to the communication module of the transceiver unit. These values are preferably transmitted in packets of three values per packet, e.g. P1, P2 and P3, forming packet P1P2P3; the next packet comprises values P2, P3 and P4 forming packet P2P3P4, and the next packet comprises values P3, P4 and P5 forming packet P3P4P5, etc. Consecutive packets are transmitted at different frequencies, i.e. packet P1P2P3 is sent at a first frequency F1, packet P2P3P4 is sent at a second frequency F2, and packet P3P4P5 is sent at a third frequency F3. Next packet would have been P4P5P6 and would have been sent at the first frequency F1, etc. This type of transmission is called a frequency hopping transmission. Thus, each pressure sensor value will then be sent three times, which increases the transmission security.
The packets received by the communication unit will then be unpacked by the second communication module in the communication unit and formatted such that the pressure values may be applied to the external device in accordance with the required signal standard, e.g. BP22 signal standard or USB standard, where they will be made available, e.g. on a display means.

As mentioned above the programmable sensor conditioner means is preferably implemented by means of a PGA309 programmable analog sensor conditioner. The PGA309 is particularly designed for resistive bridge sensor applications and contains three main gain blocks for scaling differential input bridge sensor signals. Hence, as discussed in the above, a signal representing the measured physiological variable may be adapted such that a signal in a format expected by the monitor is provided. This signal format is determined by the reference voltage supplied to the sensor signal adapting circuitry and the actual value of the signal measured by the sensor. The PGA309 can be configured for use with an internal or external voltage reference. According to the present invention, an internal reference voltage of e.g. +2,5V is supplied to the PGA309 from the energy means.

Thus, the conditioner means generates an analog output voltage signal related to the sensor signal such that the measured physiological variable, i.e. the pressure, may be retrieved by the external device.

Since each sensor element is an individual item with its own characteristics, each sensor assembly comprises a calibration means, preferably an electrically erasable programmable read-only memory (EEPROM) which contains individual calibration data obtained during calibration of the sensor element performed for each individual sensor wire assembly. The calibration is performed in connection with manufacture of the pressure sensor wire. Calibration data takes into account parameters such as voltage offsets and temperature drift, etc.

The bridge pressure sensor is preferably energized from the PGA309 via an excitation voltage V_{EXC}, generated by the PGA309 circuit. As an alternative the pressure sensor may be energized from a separate energy source, e.g. a battery or a capacitor means.

For a given excitation voltage V_{EXC}, e.g. generated by the PGA309 circuit, the output voltage (V_{IN1} - V_{IN2}) of the bridge is a voltage proportional to the pressure applied to the sensor. Hence, the sensor output voltage (V_{IN1} - V_{IN2}) (sensor signal in figure 4) of the bridge is proportional to the pressure applied to the sensor, which for a given pressure will vary with the applied excitation voltage. This sensor output voltage is preferably compensated for temperature variation at the site of the sensor and is applied to the PGA309 circuit. The PGA309 circuit also includes gain blocks for adjusting the output signal from that circuit and used in addition to the output amplifying means mentioned above.

According to another preferred embodiment a processing means, preferably a microprocessor (e.g. a PIC16C770 or a nRF24E1, shown with dashed lines in figure 4) may further be employed to process and adapt the analog output voltage V_{OUT} of the conditioned sensor, which output voltage is supplied via the PGA309 programmable analog sensor conditioner. The analog output signal from the PGA309 circuit is A/D-converted prior it is applied to the processing means. To adapt the sensor signal to the BP22 signal standard, it may be necessary to process the sensor signal further before it is applied to the physiology monitor. For instance a multiplying digital-analog converter (DAC) which possibly is comprised in the processing means is supplied with digital data (e.g. a 12-bit word) representing the signal measured by the sensor element and the reference voltage. The resulting product is wirelessly sent (after having been filtered) to the external device and is proportional to the measured sensor signal and the reference voltage.

In the preferred embodiment that has been described herein the adaptation of the sensor signal to the standard, e.g. BP22 signal standard, is performed in the transceiver unit, and in particular in the sensor signal adapting circuitry. However, this adaptation, in its entirety or only in parts, may, as an alternative, instead be performed by a corresponding circuitry arranged in the communication unit. This embodiment is schematically illustrated in figure 5. The wirelessly transmitted pressure sensor values would then be in the form of "raw" measured data that would be conditioned by a processing and conditioning means in the communication unit in order to be in a correct format to be supplied to the external system according to a prescribed standard format.

The present invention is not limited to the above-described preferred embodiments. Various alternatives, modifications and equivalents may be used. Therefore, the above embodiments should not be taken as limiting the scope of the invention, which is defined by the appended claims.

## Claims

1. Transceiver unit adapted to be connected to a proximal end of a pressure sensor wire,
said transceiver unit is adapted to wirelessly communicate by a communication signal with a communication unit,
wherein the transceiver unit further comprises a sensor signal adapting circuitry and a communication module,
wherein the sensor signal adapting circuitry is adapted to receive a pressure sensor signal from said pressure sensor wire, automatically generate an output signal, related to the sensor signal, and provide the output signal to said communication module,
wherein the communication module is adapted to generate said communication signal and transfer said communication signal to the communication unit as a data stream, and
**characterized** in that the transceiver unit is configured to be fixated to the pressure sensor wire such that the transceiver unit is adapted to be used as a handle when guiding the pressure sensor wire during insertion into a patient, and such that, as the transceiver unit is rotated along a longitudinal axis of the transceiver unit, the pressure sensor wire is also rotated.

2. Transceiver unit according to claim 1, wherein said communication signal is a radio frequency signal.

3. Transceiver unit according to claim 1, wherein said data stream is in the form of data packets.

4. Transceiver unit according to claim 2, wherein said wireless radio frequency communication is performed by Bluetooth.

5. Transceiver unit according to claim 1, wherein said transceiver unit is provided with means to perform bi-directional communication with said communication unit.

6. Transceiver unit according to claim 2, wherein said radio frequency signal has a frequency of 2,4 GHz.

7. Transceiver unit according to claim 2, wherein said wireless radio frequency communication is performed by a frequency hopping technique.

8. Transceiver unit according to claim 1, wherein said communication signal is an infrared signal.

9. Transceiver unit according to claim 1, wherein said communication signal is an ultrasound signal.

10. Transceiver unit according to claim 1, wherein said communication signal is a light signal.

11. Transceiver unit according to claim 1, wherein the transceiver unit comprises an activation means configured to activate and initiate said transceiver unit.

12. Transceiver unit according to claim 11, wherein said activation means is an activation button arranged on said unit.

13. Transceiver unit according to claim 11, wherein said activation means is adapted to activate and initiate the transceiver unit when electrical connections are established between corresponding electrical contact surfaces of female and male connectors of said transceiver unit and the proximal end of the sensor wire, respectively.

14. Transceiver unit according to claim 13, wherein said transceiver unit comprises a female connector being an insulating hollow housing containing a predetermined number of hollow contact surfaces to make contact with the conductive surfaces of the male connector.

15. Transceiver unit according to claim 13, wherein at the distal end of the female connector, fastening means for securing the male connector in the female connector are provided.

16. Transceiver unit according to claim 1, wherein said transceiver unit is provided with guiding means to guide the pressure sensor wire during insertion into a patient.

17. Transceiver unit according to claim 1, wherein said transceiver unit comprises energy means to energize the transceiver unit and the circuitry of the connected pressure sensor wire.

18. Transceiver unit according to claim 1, wherein said sensor signal adapting circuitry is adapted to filter, process, and format the signal received from the pressure sensor wire.

19. Transceiver unit according to claim 1, wherein said transceiver unit is sterilizable.

20. Pressure measurement system comprising a pressure sensor wire with a pressure sensor to measure pressure inside a patient, and to provide measured pressure data to an external device,
**characterized** by further comprising
a transceiver unit according to any of claims 1-19, and
a communication unit, adapted to wirelessly communicate with said transceiver unit, said communication unit being adapted to receive said communication signal as a data stream and transfer measured pressure data to the external device, and adapted to be connected to a standard input/output connector of the external device to communicate in accordance with an established standard,
wherein the pressure sensor wire is adapted to be connected, at its proximal end, to said transceiver unit that is adapted to wirelessly communicate via said communication signal with said communication unit, arranged in connection with the external device, in order to transfer measured pressure data to the external device.

## Patentansprüche

1. Sender-Empfänger-Einheit, die zur Verbindung mit einem proximalen Ende eines Drucksensordrahts angepasst ist,
wobei diese Sender-Empfänger-Einheit angepasst ist, um drahtlos über ein Kommunikationssignal mit einer Kommunikationseinheit zu kommunizieren, wobei die Sender-Empfänger-Einheit ferner ein Sensorsignalanpassungsschaltsystem und ein Kommunikationsmodul aufweist,
wobei das Sensorsignalanpassungsschaltsystem angepasst ist, um ein Drucksensorsignal von diesem Drucksensordraht zu empfangen, automatisch ein Ausgabesignal, das in Bezug zu dem Sensorsignal steht, zu erzeugen und das Ausgabesignal diesem Kommunikationsmodul bereitzustellen,
wobei das Kommunikationsmodul angepasst ist, um dieses Kommunikationssignal zu erzeugen und dieses Kommunikationssignal zu der Kommunikationseinheit als einen Datenstrom zu übertragen, und **dadurch gekennzeichnet, dass** die Sender-Empfänger-Einheit konfiguriert ist, um an dem Drucksensordraht befestigt zu werden, so dass die Sender-Empfänger-Einheit angepasst ist, um als ein Griff verwendet zu werden, wenn der Drucksensordraht während der Einführung in einen Patienten geführt wird, und so dass, wenn die Sender-Empfänger-Einheit um die Längsachse der Sender-Empfänger-Einheit rotiert wird, der Drucksensordraht auch rotiert wird.

2. Sender-Empfänger-Einheit gemäß Anspruch 1, wobei dieses Kommunikationssignal ein Radiofrequenzsignal ist.

3. Sender-Empfänger-Einheit gemäß Anspruch 1, wobei dieser Datenstrom in Form von Datenpaketen vorliegt.

4. Sender-Empfänger-Einheit gemäß Anspruch 2, wobei diese drahtlose Radiofrequenzkommunikation über Bluetooth durchgeführt wird.

5. Sender-Empfänger-Einheit gemäß Anspruch 1, wobei diese Sender-Empfänger-Einheit mit Mitteln versehen ist, um bidirektionale Kommunikation mit dieser Kommunikationseinheit durchzuführen.

6. Sender-Empfänger-Einheit gemäß Anspruch 2, wobei dieses Radiofrequenzsignal eine Frequenz von 2,4 GHz hat.

7. Sender-Empfänger-Einheit gemäß Anspruch 2, wobei diese drahtlose Radiofrequenzkommunikation durch eine Frequenzsprungtechnik durchgeführt wird.

8. Sender-Empfänger-Einheit gemäß Anspruch 1, wobei dieses Kommunikationssignal ein Infrarotsignal ist.

9. Sender-Empfänger-Einheit gemäß Anspruch 1, wobei dieses Kommunikationssignal ein Ultraschallsignal ist.

10. Sender-Empfänger-Einheit gemäß Anspruch 1, wobei dieses Kommunikationssignal ein Lichtsignal ist.

11. Sender-Empfänger-Einheit gemäß Anspruch 1, wobei die Sender-Empfänger-Einheit Aktivierungsmittel aufweist, die konfiguriert sind, um diese Sender-Empfänger-Einheit zu aktivieren und initiieren.

12. Sender-Empfänger-Einheit gemäß Anspruch 11, wobei diese Aktivierungsmittel ein Aktivierungsknopf sind, der an dieser Einheit angeordnet ist.

13. Sender-Empfänger-Einheit gemäß Anspruch 11, wobei diese Aktivierungsmittel angepasst sind, um die Sender-Empfänger-Einheit zu aktivieren und initiieren, wenn elektrische Verbindungen zwischen entsprechenden elektrischen Kontaktflächen von Buchse und Stecker dieser Sender-Empfänger-Einheit und dem proximalen Ende des Sensordrahts hergestellt sind.

14. Sender-Empfänger-Einheit gemäß Anspruch 13, wobei diese Sender-Empfänger-Einheit eine Buchse aufweist, die ein isolierendes hohles Gehäuse ist, das eine vorbestimmte Anzahl von hohlen Kontaktflächen enthält, um Kontakt mit den leitenden Oberflächen des Steckers herzustellen.

15. Sender-Empfänger-Einheit gemäß Anspruch 13, wobei an dem distalen Ende der Buchse Befestigungsmittel zum Sichern des Steckers in der Buchse bereitgestellt sind.

16. Sender-Empfänger-Einheit gemäß Anspruch 1, wobei diese Sender-Empfänger-Einheit mit Führungsmitteln versehen ist, um den Drucksensordraht während der Einführung in einen Patienten zu führen.

17. Sender-Empfänger-Einheit gemäß Anspruch 1, wobei diese Sender-Empfänger-Einheit Energiemittel aufweist, um die Sender-Empfänger-Einheit und das Schaltsystem des verbundenen Drucksensordrahts mit Energie zu versorgen.

18. Sender-Empfänger-Einheit gemäß Anspruch 1, wobei dieses Sensorsignalanpassungsschaltsystem angepasst ist, um das Signal, das von dem Drucksensordraht empfangen wird, zu filtern, zu verarbeiten und zu formatieren.

19. Sender-Empfänger-Einheit gemäß Anspruch 1, wobei diese Sender-Empfänger-Einheit sterilisierbar ist.

20. Druckmesssystem, das einen Drucksensordraht mit einem Drucksensor aufweist, um Druck im Inneren eines Patienten zu messen und gemessene Druckdaten an ein externes Gerät zu liefern, **gekennzeichnet dadurch**, dass es ferner aufweist:
eine Sender-Empfänger-Einheit gemäß einem der Ansprüche 1-19 und
eine Kommunikationseinheit, die angepasst ist, um drahtlos mit dieser Sender-Empfänger-Einheit zu kommunizieren, wobei diese Kommunikationseinheit angepasst ist, um dieses Kommunikationssignal als einen Datenstrom zu empfangen und gemessene Druckdaten an das externe Gerät zu übertragen, und angepasst ist, um mit einem üblichen Input/Output-Konnektor des externen Geräts verbunden zu werden, um gemäß einem etablierten Standard zu kommunizieren,
wobei der Drucksensordraht angepasst ist, um mit seinem proximalen Ende mit dieser Sender-Empfänger-Einheit verbunden zu werden, die angepasst ist, um drahtlos über dieses Kommunikationssignal mit dieser Kommunikationseinheit, die in Verbindung mit dem externen Gerät angeordnet ist, zu kommunizieren, um gemessene Druckdaten an das externe Gerät zu übertragen.

## Revendications

1. Unité d'émetteur-récepteur adaptée pour être connectée à une extrémité proximale d'un fil de capteur de pression,
ladite unité d'émetteur-récepteur est adaptée pour communiquer de manière sans fil par un signal de communication avec une unité de communication,
dans laquelle l'unité d'émetteur-récepteur comprend en outre un ensemble de circuits d'adaptation de signal de capteur et un module de communication,
dans laquelle l'ensemble de circuits d'adaptation de signal de capteur est adapté pour recevoir un signal de capteur de pression en provenance dudit fil de capteur de pression, générer automatiquement un signal de sortie, lié au signal de capteur, et fournir le signal de sortie audit module de communication,
dans laquelle le module de communication est adapté pour générer ledit signal de communication et transférer ledit signal de communication à l'unité de communication comme un flux de données, et
**caractérisée en ce que** l'unité d'émetteur-récepteur est configurée pour être fixée au fil de capteur de pression de sorte que l'unité d'émetteur-récepteur soit adaptée pour être utilisée comme une poignée lors du guidage du fil de capteur de pression pendant l'insertion dans un patient, et de sorte que, à mesure que l'unité d'émetteur-récepteur est tournée le long d'un axe longitudinal de l'unité d'émetteur-récepteur, le fil de capteur de pression soit également tourné.

2. Unité d'émetteur-récepteur selon la revendication 1, dans laquelle ledit signal de communication est un signal radiofréquence.

3. Unité d'émetteur-récepteur selon la revendication 1, dans laquelle ledit flux de données est sous la forme de paquets de données.

4. Unité d'émetteur-récepteur selon la revendication 2, dans laquelle ladite communication radiofréquence sans fil est effectuée par Bluetooth.

5. Unité d'émetteur-récepteur selon la revendication 1, dans laquelle ladite unité d'émetteur-récepteur est pourvue de moyens pour effectuer une communication bidirectionnelle avec ladite unité de communication.

6. Unité d'émetteur-récepteur selon la revendication 2, dans laquelle ledit signal radiofréquence a une fréquence de 2,4 GHz.

7. Unité d'émetteur-récepteur selon la revendication 2, dans laquelle ladite communication radiofréquence sans fil est effectuée par une technique de saut de fréquence.

8. Unité d'émetteur-récepteur selon la revendication 1, dans laquelle ledit signal de communication est un signal infrarouge.

9. Unité d'émetteur-récepteur selon la revendication 1, dans laquelle ledit signal de communication est un signal ultrasonore.

10. Unité d'émetteur-récepteur selon la revendication 1, dans laquelle ledit signal de communication est un signal lumineux.

11. Unité d'émetteur-récepteur selon la revendication 1, dans laquelle l'unité d'émetteur-récepteur comprend un moyen d'activation pour activer et déclencher ladite unité d'émetteur-récepteur.

12. Unité d'émetteur-récepteur selon la revendication 11, dans laquelle ledit moyen d'activation est un bouton d'activation agencé sur ladite unité.

13. Unité d'émetteur-récepteur selon la revendication 11, dans laquelle ledit moyen d'activation est adapté pour activer et déclencher l'unité d'émetteur-récepteur lorsque des connexions électriques sont établies entre des surfaces de contact électriques correspondantes de connecteurs femelle et mâle de ladite unité d'émetteur-récepteur et l'extrémité proximale du fil de capteur, respectivement.

14. Unité d'émetteur-récepteur selon la revendication 13, dans laquelle ladite unité d'émetteur-récepteur comprend un connecteur femelle qui est un boîtier creux isolant contenant un nombre prédéterminé de surfaces de contact creuses pour établir le contact avec les surfaces conductrices du connecteur mâle.

15. Unité d'émetteur-récepteur selon la revendication 13, dans laquelle à l'extrémité distale du connecteur femelle, des moyens d'attache pour assujettir le connecteur mâle dans le connecteur femelle sont fournis.

16. Unité d'émetteur-récepteur selon la revendication 1, dans laquelle ladite unité d'émetteur-récepteur est fournie avec des moyens de guidage pour guider le fil de capteur de pression pendant l'insertion dans un patient.

17. Unité d'émetteur-récepteur selon la revendication 1, dans laquelle ladite unité d'émetteur-récepteur comprend des moyens d'énergie pour alimenter en énergie l'unité d'émetteur-récepteur et l'ensemble de circuits du fil de capteur de pression connecté.

18. Unité d'émetteur-récepteur selon la revendication 1, dans laquelle ledit ensemble de circuits d'adaptation de signal de capteur est adapté pour filtrer, traiter, et formater le signal reçu du fil de capteur de pression.

19. Unité d'émetteur-récepteur selon la revendication 1, dans laquelle ladite unité d'émetteur-récepteur est stérilisable.

20. Système de mesure de pression comprenant un fil de capteur de pression avec un capteur de pression pour mesurer la pression à l'intérieur d'un patient, et pour fournir des données de pression mesurées à un dispositif externe,
**caractérisé en ce qu'**il comprend en outre
une unité d'émetteur-récepteur selon l'une quelconque des revendications 1 à 19, et
une unité de communication, adaptée pour communiquer de manière sans fil avec ladite unité d'émetteur-récepteur, ladite unité de communication étant adaptée pour recevoir ledit signal de communication comme un flux de données et transférer des données de pression mesurées au dispositif externe, et adaptée pour être connectée à un connecteur d'entrée/sortie standard du dispositif externe pour communiquer conformément à une norme établie,
dans lequel le fil de capteur de pression est adapté pour être connecté, à son extrémité proximale, à ladite unité d'émetteur-récepteur qui est adaptée pour communiquer de manière sans fil via ledit signal de communication avec ladite unité de communication, agencée en connexion avec le dispositif externe, afin de transférer des données de pression mesurées au dispositif externe.
